(19) Europäisches Patentamt
European Patent Office
Office européen des brevets

(11) **EP 2 831 541 B1**

(12) **FASCICULE DE BREVET EUROPEEN**

(45) Date de publication et mention
de la délivrance du brevet:
**28.04.2021 Bulletin 2021/17**

(21) Numéro de dépôt: **13715386.2**

(22) Date de dépôt: **27.03.2013**

(51) Int Cl.:
***G01B 21/08*** *(2006.01)*     ***G01B 11/06*** *(2006.01)*
***G01N 33/38*** *(2006.01)*     ***B07C 5/34*** *(2006.01)*
***G01N 21/90*** *(2006.01)*

(86) Numéro de dépôt international:
**PCT/FR2013/050664**

(87) Numéro de publication internationale:
**WO 2013/144509 (03.10.2013 Gazette 2013/40)**

(54) **PROCEDE ET INSTALLATION DE MESURE DE LA REPARTITION DE VERRE DANS DES RECIPIENTS**

VERFAHREN UND VORRICHTUNG ZUR MESSUNG DER GLASVERTEILUNG IN BEHÄLTERN

METHOD AND INSTALLATION FOR MEASURING THE GLASS DISTRIBUTION IN CONTAINERS

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **27.03.2012 FR 1252730**

(43) Date de publication de la demande:
**04.02.2015 Bulletin 2015/06**

(73) Titulaire: **Tiama**
**69390 Vourles (FR)**

(72) Inventeur: **BATHELET, Guillaume**
**F-69280 Marcy l'Etoile (FR)**

(74) Mandataire: **Cabinet Beau de Loménie**
**51 avenue Jean Jaurès**
**BP 7073**
**69301 Lyon Cedex 07 (FR)**

(56) Documents cités:
**EP-A1- 0 643 297       EP-A2- 0 679 883
EP-A2- 1 020 703       FR-A1- 2 751 068
US-A1- 2006 012 804**

**Description**

La présente invention concerne le domaine technique de l'inspection de récipients ou d'objets creux translucides ou transparents présentant une haute température.

L'objet de l'invention vise plus précisément l'inspection à haute cadence d'objets tels que des bouteilles ou des flacons en verre sortant d'une machine de fabrication de formage.

Dans le domaine préféré de la fabrication de récipients en verre, il est connu d'utiliser le rayonnement infrarouge émis par les récipients en sortie de la machine de formage afin de réaliser un contrôle ou une inspection en vue de déceler d'éventuels défauts sur la surface ou à l'intérieur des récipients. Tel est le cas notamment du brevet EP 0 679 883. Le contrôle de la qualité de tels récipients permet d'éliminer ceux qui présentent des défauts susceptibles d'affecter leur caractère esthétique ou plus grave, de constituer un réel danger pour les utilisateurs ultérieurs. Ainsi, il apparaît nécessaire de contrôler la qualité de la répartition d'épaisseur de tels récipients de manière à éliminer les récipients présentant des épaisseurs trop faibles ou des différences d'épaisseurs dans certaines zones susceptibles d'affecter la résistance mécanique.

De manière classique, la machine de formage est constituée de différentes cavités équipées chacune d'un moule dans lequel le récipient prend sa forme finale à haute température. En sortie de la machine de formage, les récipients sont acheminés de manière à constituer une file sur un convoyeur de transport amenant les récipients à défiler successivement dans divers postes de traitement tels que de pulvérisation et de recuit.

Il apparaît intéressant d'identifier un défaut de formage le plus tôt possible à la sortie de la machine de formage avant les divers postes de traitement de manière à pouvoir le corriger le plus tôt possible au niveau de la machine de formage. Dans l'état de la technique, diverses solutions ont été proposées pour inspecter des récipients à haute température sortant d'une machine de formage en vue de mesurer la répartition du verre de tels récipients.

Par exemple, le brevet US 3 535 522 décrit un procédé pour mesurer l'épaisseur de verre d'un récipient consistant à mesurer le rayonnement infrarouge émis par un tel récipient en sortie de la machine de formage. La mesure du rayonnement infrarouge est réalisée alors que le récipient est placé dans un four pour homogénéiser la température du récipient à une valeur déterminée. Cette technique ne permet pas de contrôler en continu les récipients et nécessite une manipulation des récipients conduisant à un procédé lent et susceptible d'entraîner des déformations des récipients.

Le brevet EP 0 643 297 décrit un dispositif permettant d'effectuer une analyse et un diagnostic sur un procédé de fabrication de produits en verre comportant un capteur sensible au rayonnement infrarouge émis par les objets sortant de la machine de formage. Ce système comporte également un dispositif de traitement numérique faisant appel à la mise en oeuvre d'un modèle de référence mathématique afin de déterminer les écarts existants dans la distribution du verre et les causes conduisant à la présence de contraintes thermiques dans le récipient.

Il apparaît en pratique difficile voire impossible, de mettre en œuvre une telle technique dans la mesure où le modèle mathématique est extrêmement complexe à mettre en œuvre puisque la mesure du rayonnement infrarouge dépend de nombreux paramètres tels que ceux énumérés à titre d'exemple non limitatif ci-après :

- l'intensité du rayonnement infrarouge émis par les récipients chauds dépend fortement de la température suivant la loi de Stefan BOLTZMAN :

$$E = sT^4$$

où

    E = Quantité totale de radiation émise par un objet en (watts m-2)
    s = la constante de Stefan BOLTZMAN = 5.67 x10-8 Watts m-2K-4
    T = la température en degrés Kelvin (K).

Ainsi, l'intensité du rayonnement infrarouge n'est pas une fonction linéaire simple de la température. De plus, elle dépend de la longueur d'onde.

Lors de l'opération de formage, la paroi du récipient est refroidie par contact avec le moule sur sa surface externe et par l'air de soufflage sur sa surface interne. Le refroidissement rapide génère des inhomogénéités thermiques importantes dans le matériau, entre les surfaces interne et externe du récipient. L'émission du rayonnement infrarouge est donc l'intégration d'une distribution thermique dans l'épaisseur du récipient. L'émission infrarouge dans chaque point de la surface est donc complexe à appréhender.

L'intensité du rayonnement infrarouge émis par les récipients chauds dépend des caractéristiques de ces récipients chauds tels que par exemple la taille, la couleur, la forme et la composition du verre.

Il doit être considéré que la distance entre le capteur infrarouge et la sortie des moules est différente d'un moule à l'autre de sorte que le temps de refroidissement pour chaque récipient chaud est différent, de sorte que les récipients chauds présentent des températures différentes lorsqu'ils passent devant le capteur infrarouge. En d'autres termes, l'intensité du rayonnement infrarouge mesurée par le capteur dépend de l'origine du moule de fabrication et plus précisément de la position de ce moule par rapport au capteur.

En sortie de la machine de formage, les récipients sont placés par glissement sur un convoyeur. Il s'ensuit une différence de positionnement des récipients sur le convoyeur par rapport au capteur de mesure infrarouge, ce qui est à même de modifier les mesures prises.

Il résulte de ce qui précède que de nombreux paramètres influencent le rayonnement infrarouge de sorte qu'un tel brevet n'apporte pas une solution pour mesurer la répartition de l'épaisseur de verre pour des récipients à haute température. Ce brevet enseigne simplement de déterminer des écarts dans la répartition du verre c'est-à-dire des valeurs relatives d'épaisseurs entre différentes régions des récipients. Ce brevet ne permet pas de mesurer en valeur absolue l'épaisseur de verre des récipients.

Selon une variante de réalisation, ce brevet prévoit la mise en œuvre d'un capteur optique permettant de faire des images des produits en verre afin d'obtenir des informations sur des déviations et/ou la distribution du verre. Les informations sont comparées avec les données obtenues à partir du capteur sensible au rayonnement infrarouge de manière à pouvoir ajuster les critères selon lesquels les données fournies par le capteur sensible au rayonnement infrarouge ont été analysées. Si la mise en œuvre de cette variante de réalisation apporte une correction aux critères utilisés, elle ne permet pas de remédier aux inconvénients inhérents à la méthode décrite dans ce brevet et rappelés ci-dessus. Aussi, cette solution ne permet pas de mesurer l'épaisseur du verre, en valeur absolue et par suite la répartition de l'épaisseur sur une zone étendue et encore moins sur tout le récipient.

Les demandes de brevets EP 1 020 703, US 2006/0012804 et FR 2 751 068 décrivent diverses techniques pour mesurer sans contact, l'épaisseur de produits en verre présentant une haute température. Ces techniques à caractère ponctuel ne sont pas adaptées pour mesurer la répartition de l'épaisseur du verre sur une grande surface de récipients défilant à haute cadence devant le poste de mesure.

En particulier, la demande de brevet EP 1 020 703 décrit un procédé et un dispositif pour mesurer l'épaisseur de paroi de récipients en verre chaud. Le dispositif comporte un moyen de détection pour mesurer une première intensité du rayonnement émis par ce récipient à une première longueur d'onde à laquelle l'intensité varie à la fois en fonction de la température au niveau des surfaces du récipient et de l'épaisseur de paroi entre ces surfaces. Le dispositif comporte un moyen de détection pour mesurer une deuxième intensité du rayonnement émis par le récipient à une deuxième longueur d'onde à laquelle l'intensité varie en fonction de la température à la surface et est indépendante de l'épaisseur de paroi entre les surfaces. Le dispositif comporte aussi un moyen de traitement configuré pour déterminer l'épaisseur de paroi entre lesdites surfaces en tant que fonction combinée des première et deuxième intensités.

La présente invention vise à remédier aux inconvénients des techniques antérieures en proposant un procédé permettant de mesurer la répartition de l'épaisseur du verre dans des récipients en verre chaud sortant de cavités de formage, le procédé étant simple à mettre en œuvre tout en permettant des mesures précises de la répartition de l'épaisseur du verre dans des récipients.

Pour atteindre un tel objectif, le procédé de mesure de la répartition de l'épaisseur du verre dans des récipients en verre à haute température sortant de cavités de formage, vise à mettre en œuvre au moins un capteur sensible au rayonnement infrarouge émis par les récipients adapté pour réaliser une image de la répartition du rayonnement infrarouge en mesurant dans la gamme proche infrarouge la répartition du rayonnement infrarouge émis par le récipient. Le procédé comporte les étapes suivantes :

- choisir au moins une zone d'inspection du récipient, de manière que pour chaque zone d'inspection, les paramètres influençant le rayonnement infrarouge autres que l'épaisseur, présentent chacun des valeurs sensiblement homogènes sur toute ladite zone d'inspection,
- mesurer, pour chaque zone d'inspection, l'épaisseur de verre du récipient en au moins un point de mesure appartenant à la zone d'inspection, à l'aide d'un système ponctuel de mesure d'épaisseur sans contact donnant des mesures absolues d'épaisseur de verre selon le système métrique, Z
- mesurer à l'aide d'un capteur sensible au rayonnement infrarouge, la répartition du rayonnement infrarouge émis par le récipient au moins dans chaque zone d'inspection,
- déterminer, pour chaque zone d'inspection, une relation entre la mesure de l'épaisseur prise au point de mesure et le rayonnement infrarouge considéré audit point de mesure pour étalonner chaque zone d'inspection, et pour chaque récipient,
- et à partir de ladite relation et de la répartition du rayonnement infrarouge considéré sur chaque zone d'inspection, à déterminer la répartition de verre du récipient sur chaque zone d'inspection.

Le procédé selon l'invention comporte également en combinaison l'une et/ou l'autre des caractéristiques additionnelles suivantes :

- mesurer l'épaisseur de verre du récipient selon une direction perpendiculaire à la surface du récipient à l'aide du système ponctuel de mesure,
- mesurer l'épaisseur de verre du récipient selon uniquement les différents points de mesure voisins situés selon une portion de la section du récipient et pour lesquels les mesures d'épaisseurs sont fiables,
- mesurer le rayonnement infrarouge émis par le récipient par sa zone d'inspection située sur la face avant du récipient,
- utiliser en tant que relation entre la mesure d'épaisseur et le rayonnement infrarouge, un modèle mathématique déterminé à partir d'un ou de plusieurs points de mesure de l'épaisseur et du rayonnement infrarouge pour chaque zone d'inspection,
- choisir les zones d'inspection de manière que pour chaque zone d'inspection, les paramètres influençant le rayonnement infrarouge autres que l'épaisseur de verre, présentent chacun des valeurs sensiblement homogènes sur toute ladite zone d'inspection,
- choisir les zones d'inspection du récipient de manière que pour chaque zone d'inspection, la forme et/ou l'état de surface du récipient soit sensiblement constante,
- choisir les zones d'inspection du récipient de manière que pour chaque zone d'inspection, la pente du récipient soit sensiblement constante,
- choisir plusieurs zones d'inspection du récipient de manière à obtenir par combinaison une représentation tridimensionnelle partielle ou totale du récipient,
- consiste à choisir une zone d'inspection identique pour tous les récipients ou une zone d'inspection qui change en fonction des cavités d'origine des récipients ou une zone d'inspection déterminée pour chaque récipient en fonction de l'analyse du rayonnement infrarouge,
- filtrer les mesures d'épaisseur de verre réalisées par le système ponctuel de mesure sans contact et les mesures de rayonnement infrarouge pour éliminer les mesures aberrantes.

Un autre objet de l'invention concerne également une installation de mesure de la répartition de l'épaisseur du verre dans des récipients en verre sortant de cavités de formage, comportant au moins un capteur sensible au rayonnement infrarouge dans la gamme proche infrarouge émis par les récipients et permettant de déterminer une répartition de rayonnement infrarouge sur au moins une zone d'inspection et relié à une unité de contrôle et de traitement. Selon l'invention, l'installation comporte au moins un système ponctuel de mesure d'épaisseur sans contact, adapté pour mesurer l'épaisseur de verre du récipient selon une direction perpendiculaire à la surface du récipient, en au moins un point de mesure appartenant à la zone d'inspection, le système ponctuel de mesure étant relié à l'unité de traitement qui comporte des moyens pour déterminer une relation entre la mesure de l'épaisseur prise au point de mesure et le rayonnement infrarouge considéré audit point de mesure, et des moyens pour déterminer à partir de ladite relation et du rayonnement infrarouge considéré sur la zone d'inspection, la répartition de l'épaisseur de verre du récipient sur la zone d'inspection L'installation selon l'invention est revendiquée dans la revendication 11.

L'installation selon l'invention comporte également en combinaison l'une et/ou l'autre des caractéristiques additionnelles suivantes :

- en tant que système ponctuel de mesure de l'épaisseur sans contact,
  un système de triangulation laser ou confocal à codage chromatique,
- un capteur sensible au rayonnement à infrarouge, comportant un objectif dont la profondeur de champ est adaptée de manière que le rayonnement infrarouge reçu de la face arrière du récipient opposée de la face avant sur laquelle la zone d'inspection est définie, soit homogène,
- une série de systèmes ponctuels de mesure d'épaisseur situés dans un plan sensiblement perpendiculaire à la direction de défilement des récipients devant lesdits systèmes ponctuels de mesure d'épaisseur, les systèmes ponctuels mesurant l'épaisseur du récipient en des points appartenant à des zones d'inspection présentant des inclinaisons différentes.

Diverses autres caractéristiques ressortent de la description faite ci-dessous en référence aux dessins annexés qui montrent, à titre d'exemples non limitatifs, des formes de réalisation de l'objet de l'invention.

La **Figure 1** est une vue schématique d'une installation de mesure associée à une machine de formage des récipients.

La **Figure 2** est une vue schématique en perspective d'une installation de mesure conforme à l'invention.

La **Figure 3** est une vue en perspective schématique de l'installation de mesure conforme à l'invention illustrant une disposition des systèmes de mesure ponctuel d'épaisseur.

La **Figure 3A** est une vue schématique explicitant le choix de la zone d'inspection pour laquelle la répartition du verre est à mesurer.

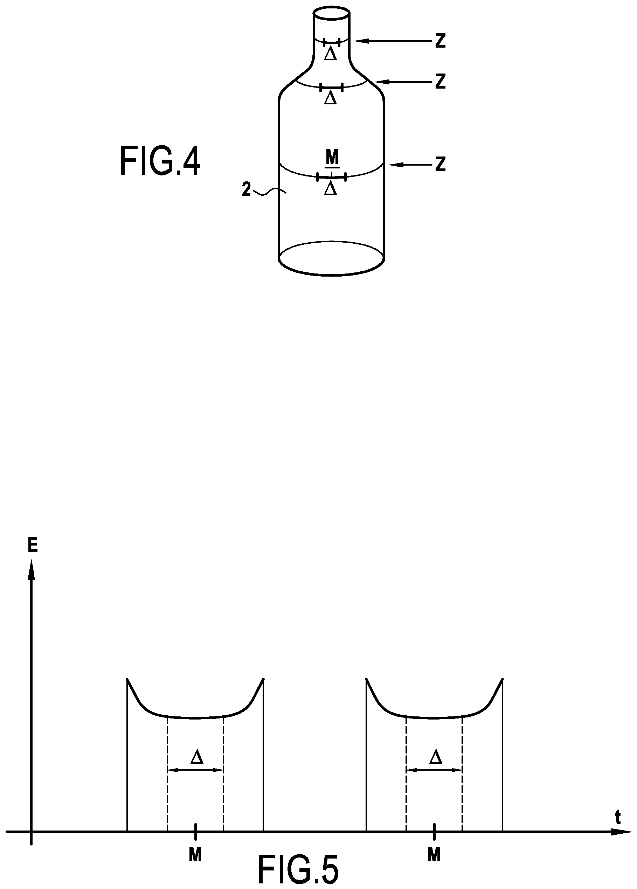

La **Figure 4** est une vue montrant l'évolution de la mesure d'épaisseur sur la circonférence d'un récipient.

La **Figure 5** est un diagramme montrant un exemple d'évolution de la mesure d'épaisseur en fonction du temps, lors du passage successif de deux récipients dans le champ de mesure d'un système de mesure ponctuel d'épaisseur.

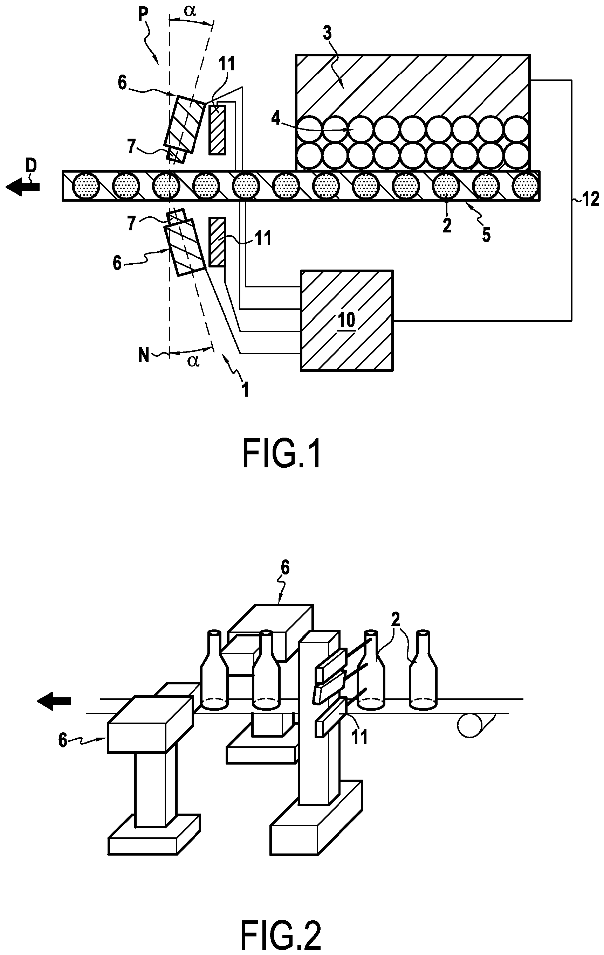

Tel que cela ressort plus précisément de la **Fig. 1**, l'objet de l'invention concerne une installation **1** permettant de mesurer la répartition de l'épaisseur de verre dans des récipients en verre **2** tels que des bouteilles ou des flacons par exemple. L'installation **1** est placée de manière à permettre d'effectuer des mesures alors que le récipient **2** sortant d'une machine de fabrication ou de formage **3** présente une haute température à savoir par exemple une température comprise entre 450 °C et 550°C.

La machine de formage **3** comporte de manière classique une série de cavités **4** assurant chacune le formage d'un récipient **2**. De manière connue, les récipients **2** qui viennent d'être formés par la machine **3** sont acheminés sur un convoyeur de sortie **5** de manière que les récipients **2** constituent une file sur le convoyeur **5** se déplaçant selon le sens **D**. Les récipients **2** sont ainsi acheminés successivement à différents postes de traitement et en particulier à l'installation de mesure **1** qui est placée au plus près de la machine de formage **3**.

L'installation **1** comporte au moins un et dans l'exemple illustré deux capteurs **6** sensibles au rayonnement infrarouge émis par les récipients **2** défilant devant chaque capteur **6**. Les capteurs **6** sont placés ainsi en sortie de la machine de formage **3** de manière à être sensible aux radiations infrarouges émises par les récipients **2**. Dans l'exemple illustré, les deux capteurs **6** sont disposés de part et d'autre du convoyeur **5** pour permettre d'inspecter les deux côtés des récipients **2**. Par exemple, chaque capteur **6** est constitué par une caméra infrarouge munie d'un objectif **7**. Selon une variante avantageuse de réalisation, la direction d'inspection de chaque capteur **6** fait un angle $\alpha$ avec la direction perpendiculaire **N** à la direction de translation **D**. Selon l'invention, le capteur d'image infrarouge est sensible dans la gamme proche infrarouge.

Les capteurs **6** sont reliés à une unité **10** de contrôle et de traitement des signaux de sortie délivrés par les capteurs **6**. Chaque capteur **6** génère un signal de sortie par exemple vidéo en réponse au rayonnement infrarouge émis par un récipient **2**. L'unité **10** est adaptée pour piloter le fonctionnement des capteurs 6 au passage d'un récipient **2** dans leur champ de vision de manière que chaque capteur **6** prenne au moins une image de chacun des récipients **2** défilant à haute cadence. L'unité **10** est adaptée pour réaliser à partir des signaux délivrés par les capteurs **6**, des images de la répartition du rayonnement infrarouge émis par les récipients **2**.

Selon une variante avantageuse de réalisation, les images prises par les capteurs **6** sensibles au rayonnement infrarouge sont filtrées pour éliminer les valeurs localement aberrantes correspondant à des particularités des récipients tels que gravures ou défauts localisés (accidents de surface, corps étranger, bouillon).

L'installation **1** selon l'invention comporte également au moins un et dans l'exemple illustré six systèmes **11** assurant une mesure sans contact de l'épaisseur du verre du récipient **2**, selon une zone ponctuelle ou localisée schématisée sur les dessins par le point **M**. Les systèmes de mesure **11** considérés comme ponctuels dans la suite de la description sont reliés à l'unité de contrôle et de traitement **10**. Avantageusement, chaque système ponctuel de mesure **11** est positionné par rapport au récipient **2** de manière que son axe de mesure soit perpendiculaire à la surface du récipient **2**.

Chaque système ponctuel de mesure **11** est de tout type connu en soi pour permettre la mesure de l'épaisseur du verre du récipient **2** présentant une haute température. Le système ponctuel de mesure **11** est par exemple un système par triangulation laser ou confocal à codage chromatique. Un tel système **11** fonctionne généralement avec une source de lumière adaptée en fonction de la teinte des récipients **2**. Avantageusement, pour éviter que les systèmes soient perturbés par le rayonnement infrarouge émis naturellement par les récipients chauds, un filtre passe bande centré sur la couleur de la source de lumière est ajouté pour le système fonctionnant par triangulation laser tandis qu'un filtre passe bas est utilisé pour couper l'infrarouge pour le système de mesure chromatique.

Chaque système ponctuel de mesure **11** permet de donner des mesures absolues d'épaisseur du verre selon le système métrique. Ainsi, il est possible de connaître l'épaisseur du verre avec une précision par exemple de l'ordre du dixième de mm.

Selon une caractéristique avantageuse de réalisation, les mesures prises par les systèmes ponctuels de mesure d'épaisseur sont filtrées pour éliminer les mesures aberrantes correspondant soit à des particularités des récipients tels que gravures ou défauts localisés, soit à des aléas de manutention, ou enfin toute perturbation du signal d'origine électrique ou électromagnétique.

La mise en œuvre de l'installation de mesure **1** découle directement du procédé de mesure conforme à l'invention qui est décrit ci-après.

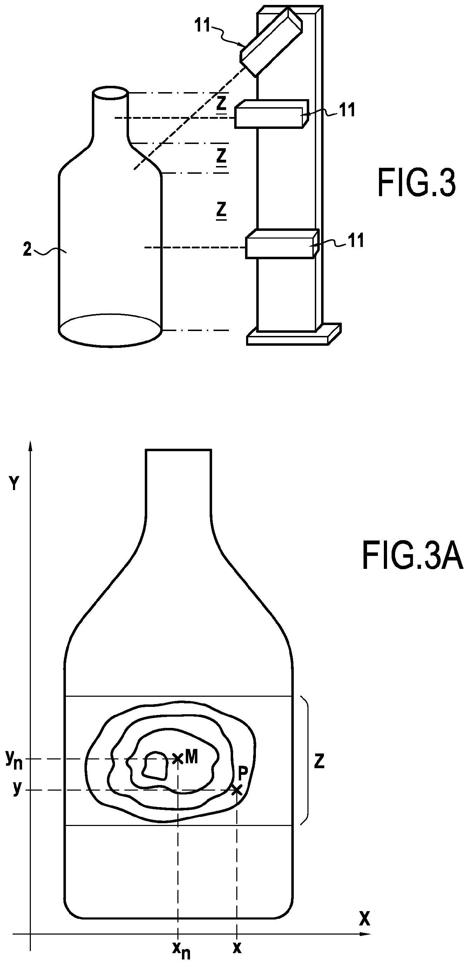

Le procédé de mesure consiste comme cela ressort plus précisément de la **Fig. 3**, à choisir au moins une zone d'inspection **Z** du récipient **2** pour laquelle la répartition d'épaisseur du verre est à mesurer. La zone d'inspection **Z** est choisie de manière que la relation d'épaisseur du verre en fonction de l'intensité du rayonnement infrarouge soit homogène sur toute cette zone d'inspection **Z**. Aussi, pour mesurer la répartition de l'épaisseur de verre sur la totalité du récipient, plusieurs zones d'inspection **Z** sont choisies.

D'une manière générale, chaque élément de surface du récipient **2** assimilé à un point P à la **Fig. 3A**, de coordonnées **x**, **y** émet un rayonnement infrarouge Ir (x, y). Il doit être considéré que le rayonnement infrarouge **Ir** (x, y) émis en chaque point **P** (x, y) et pour chaque longueur d'onde λ dépend principalement de l'épaisseur de verre E(x, y), de la température T(x, y), intégrée sur toute l'épaisseur de la paroi du récipient **2**, de l'absorption spectrale a(λ,x,y) du matériau du récipient, de la réflexion spectrale r(λ,x,y) du matériau du récipient, et de la forme et de l'état de surface $F_0$(x,y), du récipient et en particulier de l'orientation de la surface d'émission par rapport à la direction de mesure.

Pour simplifier le procédé, il peut être fait l'hypothèse que la composition du matériau est homogène dans au moins chaque zone d'inspection **Z** voire dans le récipient **2** ou tous les récipients **2**, si bien que l'absorption spectrale a(λ) et la réflexion spectrale r(λ) ne varient pas selon la position x et y.

L'épaisseur de verre E(x, y) d'un récipient est donc reliée au rayonnement infrarouge par la relation approchée (1) du type suivant :

$$E(x, y) = F(Ir(x, y)), T(x, y), F_0(x, y), a(\lambda), r(\lambda))$$

Selon l'invention, chaque zone d'inspection **Z** est choisie de manière que les paramètres influençant le rayonnement infrarouge **Ir** autres que l'épaisseur de verre présentent chacune des valeurs sensiblement homogènes sur toute ladite zone d'inspection **Z**. Autrement dit, à partir de la relation (1), il peut être formalisé une relation simplifiée telle que E(x, y) = f**z**(Ir(x, y)) avec f**z** la fonction simplifiée, cette relation étant constante dans toute la zone d'inspection **Z** pour laquelle chaque paramètre T(x, y), $F_0$(x,y), a(λ), r(λ) est homogène ou a priori déterminé.

Le procédé selon l'invention repose sur l'hypothèse que la relation d'épaisseur du verre en fonction de l'intensité du rayonnement infrarouge n'est pas modifiée de façon significative dans chacune des zones d'inspection **Z** puisque les autres paramètres varient de façon faible. Les fonctions simplifiées spécifiques **fz** pour chacune des zones d'inspection reposent sur un modèle mathématique issu d'une modélisation mathématique prenant en compte les lois de la physique (thermodynamique, transferts de chaleur par conduction et rayonnement, thermographie des corps semi-transparents) ou par modélisation empirique à l'aide d'abaques ou de tables expérimentales.

Cette fonction simplifiée **fz** peut être décrite par un modèle mathématique comme par exemple une équation aux dérivées partielles. Dans ce cas, ledit modèle mathématique peut être obtenu par simplification de la fonction **F**.

Selon une variante avantageuse du procédé, la fonction simplifiée **fz** peut être selon un modèle mathématique unique pour chaque zone, seuls les paramètres ou coefficients varient selon les zones. Lesdits coefficients sont alors calculés pour chaque récipient et pour chaque zone, à partir des mesures d'épaisseur au point de mesure **M**.

Selon une autre variante du procédé, la fonction **fz** peut avoir une expression différente pour chaque zone, par exemple en fonction de la forme a priori connue du récipient dans la zone (forme conique, cylindrique, section carré). Dans ce cas, la fonction **fz** est différente pour chaque zone, mais néanmoins, ses paramètres sont recalculés pour chaque récipient à partir des mesures d'épaisseur au point de mesure **M**.

Selon une troisième variante, la fonction simplifiée **fz** est purement empirique et représentée par des abaques obtenus par l'expérience. Dans ce cas, les mesures ponctuelles d'épaisseur servent à sélectionner des points de fonctionnement sur lesdits abaques, et la relation entre épaisseur et rayonnement découle du parcours des abaques ainsi choisis.

Selon une autre variante, la fonction **fz** est obtenue à partir des mesures d'épaisseurs de plusieurs points **M** répartis selon une zone Δ parcourue par le capteur d'épaisseur durant le déplacement des articles, ce qui permet soit de fiabiliser la mesure d'épaisseur en moyennant les mesures E(**M**) soit encore d'identifier plusieurs paramètres de la fonction simplifiée **fz** lorsque celle-ci est d'ordre élevé.

Ainsi, selon les variantes précédentes, la fonction simplifiée **fz** qui est considérée comme constante dans toute une zone d'inspection **Z** est susceptible d'être différente pour une autre zone d'inspection **Z** de sorte que la fonction simplifiée **fz** est spécifique pour chacune des zones d'inspection **Z**.

Il ressort de la description qui précède que les zones d'inspection **Z** du récipient **2** sont choisies de manière que pour chaque zone d'inspection **Z**, la forme et/ou l'état de surface du récipient **2** soient sensiblement constants. Selon une variante avantageuse de réalisation, les zones d'inspection **Z** du récipient **2** sont choisies de manière que chaque zone d'inspection **Z** corresponde à une région de la paroi du récipient **2** dont la pente est sensiblement constante.

Si la mesure de la répartition de l'épaisseur du verre doit être effectuée sur toute la hauteur du récipient **2**, alors le nombre de zones d'inspection **2** est lié dans le cas particulier, au nombre de pentes que présente la paroi du récipient **2**. Dans l'exemple illustré, le récipient **2** présente trois zones d'inspection **Z** correspondant au corps vertical du récipient, à l'épaule du récipient présentant une plus faible pente et au goulot du récipient **2** présentant à nouveau une forte pente. De façon avantageuse, plusieurs zones d'inspection **Z** sont choisies de manière à obtenir par combinaison, une représentation tridimensionnelle partielle ou totale du récipient **2**. Dans l'exemple illustré, la décomposition du récipient en trois zones, inspectées de part et d'autre du convoyeur **5**, permet d'obtenir par combinaison une représentation tridimensionnelle complète de chaque récipient **2**.

Pour chaque zone d'inspection **Z**, l'épaisseur de verre du récipient **2** est mesurée à l'aide d'un système ponctuel de mesure d'épaisseur **11** en un point de mesure **M** appartement à ladite zone de mesure **Z**. De préférence, le point de mesure **M** est choisi au centre de la zone de mesure. Selon une caractéristique avantageuse de l'invention, l'épaisseur du verre est mesurée à l'aide du système ponctuel de mesure d'épaisseur **11**, selon une direction perpendiculaire à la surface du récipient **2**. Ainsi, dans le cas où le récipient **2** présente un profil avec trois pentes différentes, l'installation comporte au moins trois systèmes ponctuels de mesure **11**. Cependant dans l'exemple illustré, le récipient **2** défile en translation devant l'installation de mesure **1** de sorte que pour la mesure de la répartition de l'épaisseur sur l'ensemble du récipient, il est prévu de disposer les systèmes ponctuels de mesure d'épaisseur **11** de part et d'autre du plan de défilement des récipients. Dans l'exemple illustré, trois systèmes ponctuels de mesure d'épaisseur **11** sont disposés de chaque côté du convoyeur de défilement **5** pour mesurer l'épaisseur respectivement du goulot, de l'épaule et du corps du récipient **2**.

Comme expliqué ci-dessus, chaque récipient **2** défile devant le système ponctuel de mesure **11** de sorte que la mesure d'épaisseur **E** évolue en fonction de la position du récipient **2** par rapport au système ponctuel de mesure **11** (**Fig**. 4 et **5**). L'unité de contrôle et de traitement **10** est adaptée pour éliminer les mesures aberrantes de l'épaisseur **E** qui apparaissent au-delà d'un angle déterminé entre la direction de mesure du système ponctuel de mesure **11** et la normale à la surface du récipient **2**. L'unité de contrôle et de traitement **10** permet de déterminer, à partir des mesures d'épaisseur **E**, la position du point **M** de la surface du récipient dont de préférence, la normale est confondue à la direction de mesure du système ponctuel de mesure **11**. Bien entendu, l'unité de contrôle et de traitement **10** est à même de prendre en compte les mesures d'épaisseurs en différents points voisins du point **M**, situés selon une portion △ de la section du récipient **2** et pour lesquels les mesures d'épaisseurs sont fiables en raison de l'orthogonalité de la surface à la direction de mesure et/ou de la présence de la paroi mesurée dans la zone de mesure du capteur, et/ou l'absence d'artefacts ou défaut ou gravures.

Il doit être considéré que les mesures d'épaisseurs instantanées sont considérées comme étant ponctuelles dans le sens où la zone de mesure est petite par rapport à la zone d'inspection **Z**. Par exemple, la zone de mesure de l'épaisseur du verre correspond à la taille du pixel de l'image de la répartition du rayonnement infrarouge, à savoir par exemple inférieure à 1 mm$^2$ pour un récipient **2** de 100 mm de diamètre.

Le procédé selon l'invention consiste également à mesurer à l'aide du capteur sensible au rayonnement infrarouge **6**, le rayonnement infrarouge émis par chaque récipient **2** dans chacune des zones d'inspection **Z** choisies.

Le procédé consiste à déterminer pour chaque récipient **2** et pour chaque zone d'inspection **Z**, une relation entre la mesure de l'épaisseur **E** prise au point de mesure **M**, soit E(M), et le rayonnement infrarouge considéré audit point de mesure **M**, soit Ir(M). En d'autres termes, la mesure d'épaisseur délivrée par les capteurs ponctuels de mesure **11** sert à étalonner chaque zone d'inspection **Z** et pour chaque récipient **2**. A partir de cette relation E=fz(Ir) et de la répartition du rayonnement infrarouge Ir(x,y) considéré sur l'étendue de chaque zone d'inspection **Z**, le procédé permet de déterminer la répartition de l'épaisseur (E(x,y)) du récipient sur ladite zone d'inspection **Z**.

Une telle méthode permet ainsi de déterminer avec précision la répartition de l'épaisseur du verre pour chaque récipient **2**. Cette méthode permet notamment de s'affranchir :

- de la teinte du verre puisque la transmission et les variations de teinte du verre sont prise en compte par l'étalonnage au travers de la mesure d'épaisseur,
- de l'orientation des récipients puisque les mesures d'épaisseur et de rayonnement infrarouge sont synchronisés,
- des variations de température dans le temps des récipients **2** dans la mesure où chaque zone d'inspection est sélectionnée pour être homogène en température et où la fonction E=fz(Ir) ou au moins ses paramètres sont recalculés pour chaque article. Ce découpage en zones d'inspection **Z** peut résulter d'une expérience et/ou d'une modélisation mathématique, d'une mesure par échantillonnage ou en continu, à l'aide de capteurs de température (pyromètre ou caméra thermographique).

Selon une autre caractéristique avantageuse de réalisation, le procédé consiste à mesurer la répartition du rayonnement infrarouge émis par une zone d'inspection **Z** située sur la face avant du récipient **2**. A cet effet, chaque capteur sensible au rayonnement infrarouge **6** comporte un objectif **7** dont la profondeur de champ est adaptée de manière que la répartition du rayonnement infrarouge de la face arrière du récipient opposée de la face avant sur laquelle la zone d'inspection est définie soit uniforme. En d'autres termes, l'erreur de mesure susceptible d'être générée par les variations de rayonnement issues de la face arrière du récipient **2** est éliminée.

Selon une variante de réalisation, la zone d'inspection **Z** du récipient pris en compte est identique pour tous les récipients identiques d'une même production. Selon un autre mode de réalisation, la zone d'inspection **Z** prise en compte change en fonction des cavités d'origine **4** des récipients. Ainsi, la même zone d'inspection **Z** peut être prise en compte pour tous les récipients provenant de la même cavité d'origine **4**. Selon un autre mode de réalisation, la zone d'inspection **Z** est déterminée pour chaque récipient en fonction de l'analyse du rayonnement infrarouge.

L'invention n'est pas limitée aux exemples décrits et représentés car diverses modifications peuvent y être apportées sans sortir de son cadre tel que spécifié dans les revendications 1 à 14.

**Revendications**

1. Procédé de mesure de la répartition de l'épaisseur du verre dans des récipients(**2**) en verre à haute température sortant de cavités de formage (**4**), le procédé mettant en oeuvre au moins un capteur sensible au rayonnement infrarouge (**6**) émis par les récipients (**2**) adapté pour réaliser une image de la répartition du rayonnement infrarouge en mesurant dans la gamme proche infrarouge la répartition du rayonnement infrarouge émis par le récipient, **caractérisé en ce qu'**il comporte pour chaque récipient, les étapes suivantes :

   - choisir au moins une zone d'inspection (**Z**) du récipient (**2**) de manière que pour chaque zone d'inspection (**Z**), les paramètres influençant le rayonnement infrarouge autres que l'épaisseur de verre, présentent chacun des valeurs sensiblement homogènes sur toute ladite zone d'inspection (**Z**),
   - mesurer, pour chaque zone d'inspection (**Z**), l'épaisseur de verre du récipient en au moins un point de mesure appartenant à la zone d'inspection (**Z**), à l'aide d'un système ponctuel de mesure d'épaisseur sans contact (**11**), donnant des mesures absolues d'épaisseur de verre selon le système métrique,
   - mesurer à l'aide du capteur sensible au rayonnement infrarouge (**6**), la répartition du rayonnement infrarouge émis par le récipient (**2**) au moins dans chaque zone d'inspection (**Z**),
   - déterminer, pour chaque zone d'inspection (**Z**), une relation entre la mesure de l'épaisseur prise au point de mesure et le rayonnement infrarouge considéré audit point de mesure pour étalonner chaque zone d'inspection (**Z**), et pour chaque récipient (**2**),
   - et à partir de ladite relation et de la répartition du rayonnement infrarouge considéré sur chaque zone d'inspection, à déterminer la répartition de l'épaisseur de verre du récipient sur chaque zone d'inspection (**Z**).

2. Procédé selon la revendication 1, **caractérisé en ce qu'**il consiste à mesurer l'épaisseur de verre du récipient (**2**) selon une direction perpendiculaire à la surface du récipient à l'aide du système ponctuel de mesure.

3. Procédé selon la revendication 1 ou 2, **caractérisé en ce qu'**il consiste à mesurer l'épaisseur de verre du récipient (**2**) selon uniquement les différents points de mesure voisins situés selon une portion ($\Delta$) de la section du récipient (**2**) et pour lesquels les mesures d'épaisseurs sont fiables.

4. Procédé selon la revendication 1, **caractérisé en ce qu'**il consiste à mesurer le rayonnement infrarouge émis par le récipient (**2**) par sa zone d'inspection (**Z**) située sur la face avant du récipient prise par rapport au capteur sensible au rayonnement infrarouge (**6**).

5. Procédé selon la revendication 1, **caractérisé en ce qu'**il consiste à utiliser en tant que relation entre la mesure d'épaisseur et le rayonnement infrarouge, un modèle mathématique déterminé à partir d'un ou de plusieurs points de mesure de l'épaisseur et du rayonnement infrarouge pour chaque zone d'inspection (**Z**).

6. Procédé selon l'une des revendications 1 à 5, **caractérisé en ce qu'**il consiste à choisir les zones d'inspection (**Z**) du récipient de manière que pour chaque zone d'inspection (**Z**), la forme et/ou l'état de surface du récipient soit sensiblement constante.

7. Procédé selon la revendication 6, **caractérisé en ce qu'**il consiste à choisir les zones d'inspection (**Z**) du récipient de manière que pour chaque zone d'inspection (**Z**), la pente du récipient soit sensiblement constante.

8. Procédé selon l'une des revendications 1 à 7, **caractérisé en ce qu'**il consiste à choisir plusieurs zones d'inspection (**Z**) du récipient (**2**) de manière à obtenir par combinaison une représentation tridimensionnelle partielle ou totale du récipient.

9. Procédé selon l'une des revendications 1 à 8, **caractérisé en ce qu'**il consiste à choisir une zone d'inspection (**Z**) identique pour tous les récipients ou une zone d'inspection (**Z**) qui change en fonction des cavités (**4**) d'origine des récipients ou une zone d'inspection (**Z**) déterminée pour chaque récipient en fonction de l'analyse du rayonnement infrarouge.

10. Procédé selon l'une des revendications 1 à 9, **caractérisé en ce qu'**il consiste à filtrer les mesures d'épaisseur de verre réalisées par le système ponctuel de mesure sans contact (**11**) et les mesures de rayonnement infrarouge pour éliminer les mesures aberrantes.

11. Installation de mesure de la répartition de l'épaisseur du verre dans des récipients (**2**) en verre sortant de cavités de formage (**4**), comportant au moins un capteur sensible au rayonnement infrarouge (**6**) dans la gamme proche infrarouge émis par les récipients (**2**) et permettant de déterminer une répartition de rayonnement infrarouge sur au moins une zone d'inspection (**Z**) et relié à une unité de contrôle et de traitement (**10**), **caractérisée en ce que** l'installation comporte un moyen apte à choisir au moins une zone d'inspection (**Z**) du récipient (**2**) de manière que pour chaque zone d'inspection (**Z**), les paramètres influençant le rayonnement infrarouge autres que l'épaisseur de verre, présentent des valeurs sensiblement homogènes sur toute ladite zone d'inspection (**Z**), au moins un système ponctuel de mesure d'épaisseur sans contact (**11**), adapté pour mesurer l'épaisseur de verre du récipient selon une direction perpendiculaire à la surface du récipient, en au moins un point de mesure (**P**) appartenant à la zone d'inspection (**Z**), le système ponctuel de mesure (**11**) donnant des mesures absolues d'épaisseur de verre selon le système métrique et étant relié à l'unité de traitement (**10**) qui comporte des moyens pour déterminer une relation entre la mesure de l'épaisseur prise au point de mesure et le rayonnement infrarouge considéré audit point de mesure, et des moyens pour déterminer à partir de ladite relation et du rayonnement infrarouge considéré sur la zone d'inspection, la répartition de l'épaisseur de verre du récipient sur la zone d'inspection.

12. Installation de mesure selon la revendication 11, **caractérisée en ce qu'**elle comporte en tant que système ponctuel de mesure l'épaisseur sans contact (**11**) un système de triangulation laser ou confocal à codage chromatique.

13. Installation de mesure selon la revendication 11, **caractérisée en ce que** le capteur sensible au rayonnement infrarouge (**6**) comporte un objectif (**7**) dont la profondeur de champ est adapté de manière que le rayonnement infrarouge reçu de la face arrière du récipient (**2**) opposée de la face avant sur laquelle la zone d'inspection (**Z**) est définie, soit homogène.

14. Installation de mesure selon l'une des revendications 11 à 13, **caractérisée en ce qu'**elle comporte au moins une série de systèmes ponctuels de mesure d'épaisseur (**11**) situés dans un plan sensiblement perpendiculaire à la direction de défilement des récipients devant lesdits systèmes ponctuels de mesure d'épaisseur, les systèmes ponctuels (**11**) mesurant l'épaisseur du récipient en des points appartenant à des zones d'inspection (**Z**) présentant des inclinaisons différentes.

**Patentansprüche**

1. Verfahren zum Messen der Verteilung der Dicke des Glases in Behältern (2) aus Glas bei hoher Temperatur, die aus Formgebungshohlräumen (4) austreten, wobei das Verfahren mindestens einen Sensor einsetzt, der gegenüber Infrarotstrahlung (6) empfindlich ist, die von den Behältern (2) emittiert wird, der geeignet ist, ein Bild von der Verteilung der Infrarotstrahlung durch Messen der Verteilung der Infrarotstrahlung, die von dem Behälter emittiert wird, im nahen Infrarotbereich zu erstellen, **dadurch gekennzeichnet, dass** es für jeden Behälter die folgenden Schritte aufweist:

   - Auswählen mindestens einer Inspektionszone (Z) des Behälters (2) derart, dass für jede Inspektionszone (Z) die Parameter, die die Infrarotstrahlung beeinflussen, außer der Glasdicke, jeweils im Wesentlichen homogene Werte auf der gesamten Inspektionszone (Z) aufweisen,
   - Messen für jede Inspektionszone (Z) der Dicke des Behälters an mindestens einer Messstelle, die zu der Inspektionszone (Z) gehört, mit Hilfe eines berührungslosen punktuellen Dickenmesssystems (11), das absolute Messungen der Glasdicke nach dem metrischen System gibt,
   - Messen mit Hilfe des Sensors, der gegenüber Infrarotstrahlung (6) empfindlich ist, der Verteilung der Infrarotstrahlung, die von dem Behälter (2) emittiert wird, in mindestens einer Inspektionszone (Z),
   - Bestimmen einer Beziehung zwischen der Messung der Dicke, die an der Messstelle vorgenommen wird, und der betreffenden Infrarotstrahlung an der Messstelle, um jede Inspektionszone (Z) zu kalibrieren, für jede Inspektionszone (Z) und für jeden Behälter (2),
   - und ausgehend von der Beziehung und der Verteilung der betreffenden Infrarotstrahlung in jeder Inspektionszone, Bestimmen der Verteilung der Glasdicke des Behälters in jeder Inspektionszone (Z).

2. Verfahren nach Anspruch 1, **dadurch gekennzeichnet, dass** es darin besteht, die Glasdicke des Behälters (2) in einer Richtung senkrecht zu der Oberfläche des Behälters mit Hilfe des punktuellen Messsystems zu messen.

3. Verfahren nach Anspruch 1 oder 2, **dadurch gekennzeichnet, dass** es darin besteht, die Glasdicke des Behälters (2) nur nach den verschiedenen benachbarten Messstellen, die sich entlang eines Abschnitts ($\Delta$) des Querschnitts des Behälters (2) befinden und für die die Dickenmessungen zuverlässig sind, zu messen.

4. Verfahren nach Anspruch 1, **dadurch gekennzeichnet, dass** es darin besteht, die Infrarotstrahlung, die von dem Behälter (2) emittiert wird, durch seine Inspektionszone (Z) zu messen, die sich auf der Vorderseite des Behälters befindet, die in Bezug auf den Sensor, der gegenüber Infrarotstrahlung (6) empfindlich ist, genommen wird.

5. Verfahren nach Anspruch 1, **dadurch gekennzeichnet, dass** es darin besteht, als Beziehung zwischen der Messung der Dicke und der Infrarotstrahlung ein mathematisches Modell zu verwenden, das ausgehend von einem oder mehreren Messstellen der Dicke und der Infrarotstrahlung für jede Inspektionszone (Z) bestimmt wird.

6. Verfahren nach einem der Ansprüche 1 bis 5, **dadurch gekennzeichnet, dass** es darin besteht, die Inspektionszonen (Z) des Behälters derart auszuwählen, dass für jede Inspektionszone (Z) die Form und/oder der Zustand der Oberfläche des Behälters im Wesentlichen konstant ist/sind.

7. Verfahren nach Anspruch 6, **dadurch gekennzeichnet, dass** es darin besteht, die Inspektionszonen (Z) des Behälters derart auszuwählen, dass für jede Inspektionszone (Z) die Neigung des Behälters im Wesentlichen konstant ist.

8. Verfahren nach einem der Ansprüche 1 bis 7, **dadurch gekennzeichnet, dass** es darin besteht, mehrere Inspektionszonen (Z) des Behälters (2) derart auszuwählen, um durch Kombination eine teilweise oder vollständige dreidimensionale Darstellung des Behälters zu erhalten.

9. Verfahren nach einem der Ansprüche 1 bis 8, **dadurch gekennzeichnet, dass** es darin besteht, eine identische Inspektionszone (Z) für alle Behälter oder eine Inspektionszone (Z), die sich in Abhängigkeit von den ursprünglichen Hohlräumen (4) der Behälter ändert, oder eine Inspektionszone (Z) auszuwählen, die für jeden Behälter in Abhängigkeit von der Analyse der Infrarotstrahlung bestimmt wird.

10. Verfahren nach einem der Ansprüche 1 bis 9, **dadurch gekennzeichnet, dass** es darin besteht, die Messungen der Glasdicke, die von dem berührungslosen punktuellen Dickenmesssystem (11) erstellt werden, und die Messungen der Infrarotstrahlung zu filtern, um die abweichenden Messungen zu beseitigen.

11. Vorrichtung zum Messen der Verteilung der Dicke des Glases in Behältern (2) aus Glas, die aus Formgebungshohlräumen (4) austreten, umfassend mindestens einen Sensor, der gegenüber Infrarotstrahlung (6) im nahen Infrarotbereich empfindlich ist, die von den Behältern (2) emittiert wird, und der ermöglicht, eine Verteilung der Infrarotstrahlung in mindestens einer Inspektionszone (Z) zu bestimmen, und der an eine Steuereinheit (10) angeschlossen ist,
**dadurch gekennzeichnet, dass** die Vorrichtung ein Mittel, das geeignet ist, mindestens eine Inspektionszone (Z) des Behälters (2) derart auszuwählen, dass für jede Inspektionszone (Z) die Parameter, die die Infrarotstrahlung beeinflussen, außer der Glasdicke, jeweils im Wesentlichen homogene Werte auf der gesamten Inspektionszone (Z) aufweisen, mindestens ein berührungsloses punktuelles Dickenmesssystem (11), das geeignet ist, die Glasdicke des Behälters in einer Richtung senkrecht zu der Oberfläche des Behälters an mindestens einer Messstelle (P), die zu der Inspektionszone (Z) gehört, zu messen, wobei das berührungslose punktuelle Dickenmesssystem (11) absolute Messungen der Glasdicke nach dem metrischen System gibt und an der Steuereinheit (10) angeschlossen ist, die Mittel zum Bestimmen einer Beziehung zwischen der Messung der Dicke, die an der Messstelle vorgenommen wird, und der betreffenden Infrarotstrahlung an der Messstelle aufweist, und Mittel zum Bestimmen, ausgehend von der Beziehung und der Verteilung der betreffenden Infrarotstrahlung in jeder Inspektionszone, der Verteilung der Glasdicke des Behälters der Inspektionszone aufweist.

12. Vorrichtung zum Messen nach Anspruch 11, **dadurch gekennzeichnet, dass** sie als berührungsloses punktuelles Dickenmesssystem (11) ein Lasertriangulationssystem oder Konfokalsystem mit chromatischer Kodierung aufweist.

13. Vorrichtung zum Messen nach Anspruch 11, **dadurch gekennzeichnet, dass** der Sensor, der gegenüber Infrarotstrahlung (6) empfindlich ist, ein Objektiv (7) aufweist, dessen Schärfentiefe derart geeignet ist, dass die Infrarotstrahlung, die von der Rückseite des Behälters (2) empfangen wird, die der Vorderseite gegenüberliegt, auf der die Inspektionszone (Z) definiert ist, homogen ist.

14. Vorrichtung zum Messen nach einem der Ansprüche 11 bis 13, **dadurch gekennzeichnet, dass** sie mindestens eine Reihe von punktuellen Dickenmesssystemen (11) aufweist, die in einer Ebene angeordnet sind, die im Wesentlichen senkrecht zu der Vorbeilaufrichtung der Behälter vor den punktuellen Dickenmesssystemen ist, wobei die punktuellen Systeme (11) die Dicke des Behälters an Stellen misst, die zu Inspektionszonen (Z) gehören, die verschiedene Neigungen aufweisen.

**Claims**

1. A method for measuring the distribution of the thickness of the glass in glass containers (2) at a high temperature leaving shaping cavities **(4),** the method applying at least one sensor sensitive to the infrared radiation (6) emitted by the containers (2) suitable for producing an image of the distribution of the infrared radiation, by measuring in the near infrared range the distribution of infrared radiation emitted by the container, **characterized in that** it includes for each container the following steps:

   - selecting at least one inspection area (Z) of the container (2) so that for each inspection area (Z), the parameters having an influence on the infrared radiation other than the glass thickness, each have substantially homogeneous values over the whole of said inspection area (Z),
   - measuring, for each inspection area (Z), the glass thickness of the container in at least one measurement point belonging to the inspection area (Z), by means of a contactless point-like thickness measurement system (11) giving absolute thickness measurements of the glass according to the metric system,
   - measuring by means of the sensor sensitive to infrared radiation (6), the distribution of the infrared radiation emitted by the container (2) at least in each inspection area (Z),
   - determining, for each inspection area (Z), a relationship between the measurement of the thickness taken at the measurement point and the relevant infrared radiation at said measurement point, for calibrating each inspection area (Z) and for each container (2),
   - and from said relationship and from the distribution of the relevant infrared radiation of each inspection area, determining the distribution of the glass thickness of the container of each inspection area (Z).

2. The method according to claim 1, **characterized in that** it consists of measuring the glass thickness of the container (2) along a direction perpendicular to the surface of the container by means of the point-like measurement system.

3. The method according to claim 1 or 2, **characterized in that** it consists of measuring the glass thickness of the container (2) in the different neighboring measurement points located along a portion (Δ) of the section of the container (2), for which the measurements of thicknesses are reliable.

4. The method according to claim 1, **characterized in that** it consists of measuring the infrared radiation emitted by the container (2), by each inspection area (Z) located on the front face of the container taken relatively to the sensor sensitive to infrared radiation (6).

5. The method according to claim 1, **characterized in that** it consists of using as a relationship between the thickness measurement and the infrared radiation, a mathematical model determined from one or several thickness measurement points and from the infrared radiation for each inspection area (Z).

6. The method according to one of claims 1 to 5, **characterized in that** it consists of selecting the inspection area (Z) of the container so that for each inspection area (Z), the shape and/or the surface condition of the container is substantially constant.

7. The method according to claim 6, **characterized in that** it consists of selecting the inspection areas (Z) of the container so that for each inspection area (Z) the slope of the container is substantially constant.

8. The method according to one of claims 1 to 7, **characterized in that** it consists of selecting several inspection areas (Z) of the container (2) so as to obtain by combination a partial or complete three-dimensional representation of the container.

9. The method according to one of claims 1 to 8, **characterized in that** it consists of selecting an inspection area (Z) identical for all the containers or an inspection area (Z) which changes according to the original cavities (4) of the containers or an inspection area (Z) determined for each container depending on the analysis of the infrared radiation.

10. The method according to one of claims 1 to 9, **characterized in that** it consists of filtering the glass thickness measurement conducted by the contactless point-like measurement system (**11**) and the infrared radiation measurement for suppressing the outlying measurements.

11. An installation for measuring the distribution of the thickness of the glass in glass containers (**2**) leaving shaping cavities (**4**), including at least one sensor sensitive to infrared radiation (**6**) in the near infrared range emitted by the containers (**2**) and allowing determination of a distribution of infrared radiation over at least one inspection area (**Z**) and connected to a control and processing unit (**10**), **characterized in that** the installation includes a means adapted to select at least one inspection zone (**Z**) of the container (**2**) so that for each inspection area (**Z**), the parameters influencing the infrared radiation other than the thickness of glass, have values substantially homogeneous over the whole of said inspection zone (**Z**), at least one contactless point-like thickness measurement system (**11**), suitable for measuring the glass thickness of the container along a direction perpendicular to the surface of the container, in at least one measurement point (**P**) belonging to the inspection area (**Z**), the point-like measurement system (**11**) giving absolute thickness measurements of the glass according to the metric system and being connected to the processing unit (**10**) which includes means for determining a relationship between the measurement of the thickness taken at the measurement point and the relevant infrared radiation at said measurement point, and means for determining from said relationship and from the relevant infrared radiation on the inspection area, the distribution of the glass thickness of the container all over the inspection area.

12. A measurement installation according to claim 11, **characterized in that** it includes as a contactless point-like thickness measurement system (**11**) a laser triangulation or confocal system with color coding.

13. The measurement installation according to claim 11, **characterized in that** the sensor sensitive to the infrared radiation (**6**) includes an objective (**7**) for which the field depth is adapted so that the infrared radiation received from the rear face of the container (**2**) opposite to the front face on which the inspection area (**Z**) is defined, is homogeneous.

14. The measurement installation according to one of claims 11 to 13, **characterized in that** it includes at least one series of point-like thickness measurement systems (**11**) located in a plane substantially perpendicular to the direction of the containers moving past said point-like thickness measurement systems, the point-like system (**11**) measuring the thickness of the container at points belonging to inspection areas (**Z**) having different tilts.

FIG.1

FIG.2

11

Z

Z

11

Z

11

2

**FIG.3**

Y

**FIG.3A**

$y_n$

×M

P

y

×

Z

X

$x_n$    x

FIG.4

FIG.5

## RÉFÉRENCES CITÉES DANS LA DESCRIPTION

**Documents brevets cités dans la description**

- EP 0679883 A
- US 3535522 A
- EP 0643297 A
- EP 1020703 A
- US 20060012804 A
- FR 2751068